# EUROPEAN PATENT SPECIFICATION

(11) **EP 0 433 464 B1**
(45) Date of publication and mention of the grant of the patent: **07.06.1995**
(21) Application number: 90909841.0
(22) Date of filing: 28.06.1990
(51) Int. Cl.: A61B 17/36

(54) **Laser guide probe**
Laserführungssonde
Sonde de guidage de laser

(30) Priority: 30.06.1989 JP 169029/89
(43) Date of publication of application: 26.06.1991
(73) Proprietor: S.L.T. JAPAN CO, LTD., Tokyo 102 (JP)
(72) Inventor: Daikuzono, Norio 381, Kofudai 4-chome, Chiba-ken 290-02 (JP)
(74) Representative: Bloch, Gérard
(86) International application number: PCT/JP90/00849
(87) International publication number: WO 91/00062

(56) References cited:
- GB-A- 2 182 565
- GB-A- 2 214 084
- JP-A- 6 357 041
- JP-A-62 213 748
- JP-A-63 197 447
- JP-A-63 200 750
- JP-A-63 318 934
- JP-B- 6 140 419
- JP-U-60 190 310
- JP-U-62 194 314
- JP-U-62 201 961
- JP-U-62 202 815

## Description

This invention relates to a laser light transmitting probe, which irradiates laser light against living tissues of an animal such as a human body for use an incision, vaporization of the living tissues or a thermal therapy.

Medical treatments such as incisions of living tissues of animal organisms by irradiation with laser light are conspicuous due to its ability of hemostasis in these days.

It had been the conventional method that the laser light is irradiated from the tip end of an optical fiber which is brought out of contact with the living tissues. But this method causes severe damage to the fore end portion of the optical fiber. Therefore, a method using a contact probe which has been utilized lately is as follows;

First, laser light is transmitted into an optical fiber, whose fore end portion locates adjacent to treated living tissues. Next, the laser light fed out from the optical fiber is impinged into an emitting probe, which is brought into or out of contact with the living tissues (hereafter "living tissue" is sometimes expressed by "tissue" only). Then, the laser light is emitted from the surface of the fore end portion of the probe to be irradiated against the living tissues. In this case, the probe should be brought into contact with the living tissues.

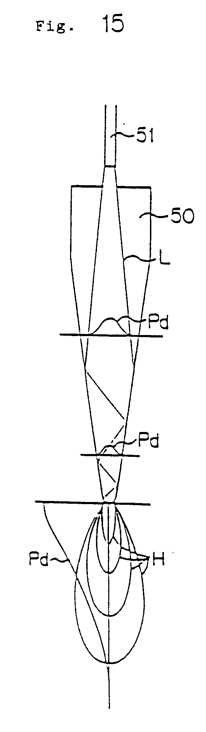

The inventor developed many kinds of contact probes which are utilized for various purposes. Fig. 15 shows a probe 50 made of sapphire, quartz and the like. Usually, its fore end portion is tapered and conical shaped uniformly.

However, referring to Fig. 15, laser light L is fed by means of an optical fiber 51 into the probe 50, which is of long and narrow conical shape with a round tip end and whose outer surface is smooth. The laser light L passing through the probe 50 is reflected and refracted on an inner surface to reach the tip end, finally is emitted from the tip end.

In this case, a power density and power distribution of the laser light L are shown as contour lines H and a curve Pd respectively in Fig. 15. Accordingly, it is obvious that the laser light L is concentratedly emitted from the tip end of the probe 50. Therefore, the effective area of the laser light irradiation is very small and exists only in living tissues adjacent to the inserting portion of the probe.

On the other hand, when medical operations are carried out, various surgical treatments such as an incision, vaporization, an exfoliation of the tissues and occasionally hemostasis for ulcerated tissues are required.

In these prior art, the exfoliation can not be carried out by the laser light irradiation, thus, only mechanical incisions have been applied. Further, when the large target area having ulcerated tissues is exfoliated, a medical cutter must be moved many times little by little.

A laser light transmitting comprising an upper part into which laser light is impinged, and a lower part from which said laser light is emitted, at least one flat surface being formed on said lower part, by partial and longitudinal cutting away of one side of the lower part and having two side edges, is already known from GB-A-2182565. This reference teaches a sharp edge at the tip end of the probe, whose function is to cut.

It is therefore the main object of the present invention to provide a laser light transmitting probe, which permits efficiently and selectively treatments such as an exfoliation and hemostasis as well as an incision and vaporization corresponding to the conditions of each target area of living tissues.

To this end, the invention relates to a probe of the above mentioned type, characterized by the fact that a surface layer, containing laser light absorbing particles and laser light scattering particles having a larger refractive index than that of the material of said probe, is formed on said at least one flat surface.

In a preferable embodiment, the lower part of the probe is provided with a conical part, while the probe is symmetric with respect to its axis.

In another preferred embodiment, the surface layer also contains a binder made from a laser light transmissible material, formed on said at least one flat surface. Further, the surface layer is more preferably formed on a rough surface covering said at least one flat surface of the probe.

Hereafter, the lower part of the probe, which is provided with at least one flat surface, is expressed by a flat part.

In still another preferable embodiment, the flat portion is bent with respect to the upper part.

According to the present invention, the energy power level of the emitted laser light is normally the highest from the tip end of the probe, lower from the both side-edges of the flat part and the lowest from the flat surface, although the level changes slightly due to the structure of the probe. Therefore, by the laser light emitted from the tip end, the tissues can be incised efficiently. In a treatment which requires a low energy level of the laser light such as hemostasis for ulcerated tissues, the laser light emitted from the flat surface can be utilized efficiently. Then, with the side-edges of the flat part, the exfoliation or so called side- incision can be performed easily. Further, by inserting the tip end or the both side-edges of the flat part into the tissues, a small target area can be treated.

Accordingly, as various types of surgical treatments can be performed with only this probe, any other special probes are not required. That is to say, this probe can be used suitably for many kinds of purposes.

In the present invention, the surface layer, which contains light scattering particles made of e.g. sapphire and the like having a larger refractive index than that of a transmissible member , which is used for forming probe as its body, is preferably formed on the transmissible member. While laser light L emitted from the transmissible member passes through the surface layer , the laser light L, which impinges on the light scattering particle , is partially reflected on the surface of the light scattering particle , or is partially penetrated into and is emitted from the particle with refraction. Therefore, the laser light L is emitted in various directions from the whole surface layer 5. This produces a large area of laser light irradiation.

Further, the surface layer contains laser light absorbing particles made of e.g. carbon and the like. Accordingly, when the laser light L impinges on the laser light absorbing particle , the greater part of the energy of the laser light L is converted to heat energy by means of the laser light absorbing particle , and the tissues are heated by the heat energy from the surface layer .

Thus, as the vaporization of the tissues is accelerated, the tissues can be incised with a low energy of the laser light penetrated into the transmissible member . Therefore, when the tissues are incised, the transmissible member can be moved rapidly. In other words, the probe can be moved rapidly. Further, high power level of the laser light is not required when the laser light is penetrating into the transmissible member . As a result, the medical operation can be carried out in short time, further with a cheap and small scaled laser light generator.

On the other hand, as for the producing method of the surface layer, for example, if a dispersion containing the laser light absorbing particles and light scattering particles is coated on the surface of the transmissible member, after a vaporization of a dispersion medium, the contact of the probe with the tissues or other substances causes a damage to the surface layer. Because the both kinds of particles are attached to the surface of the transmissible member only by physical adsorptive power. Therefore, by a binder which sticks the laser light absorbing particles and the light scattering particles to the surface of the transmissible member, an adhesion of the surface layer to the transmissible member is enhanced.

In this case, the binder is preferably made of light transmissible material such as quartz and the like to ensure the emission of the laser light from the surface layer . Laser light transmissible particles which have a melting point same to or lower than that of the transmissible member are preferably used as the transmissible material and are dispersed together with the absorbing particles and the light scattering particles in a proper liquid such as water. Then the transmissible member painted with this dispersion is baked at a temperature which is higher than a melting point of the transmissible particle and within a limit as the transmissible member can keep its shape. Accordingly, the transmissible particles as the binder are melted to form the surface layer of high mechanical strength together with the laser light absorbing particles and the light scattering particles. Therefore, the damages to the surface layer can be reduced because of its high strength.

The present invention shall be better understood with reference to the accompanying drawings.

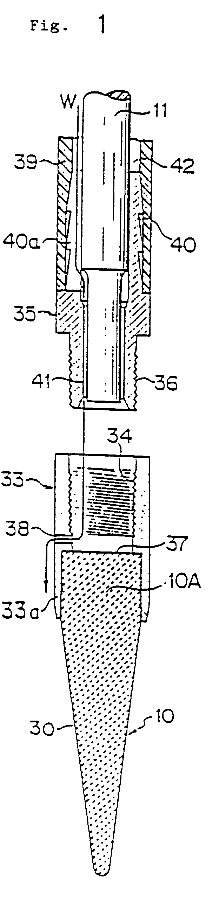

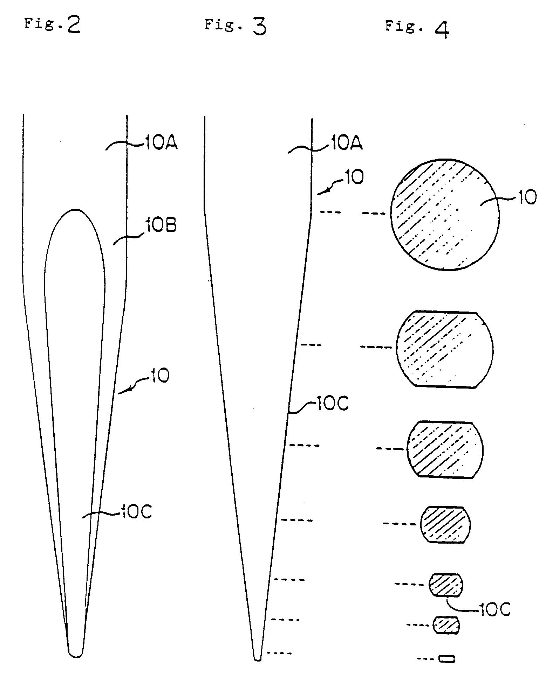

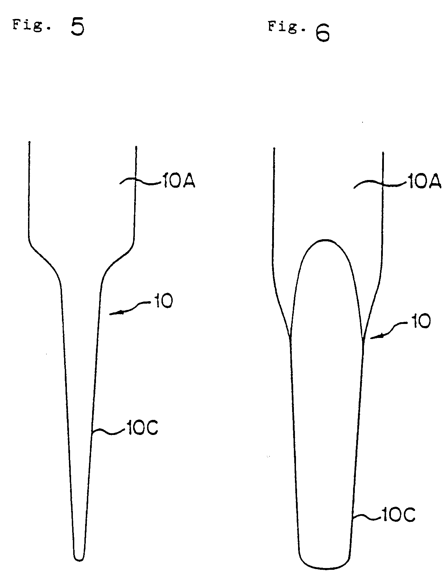

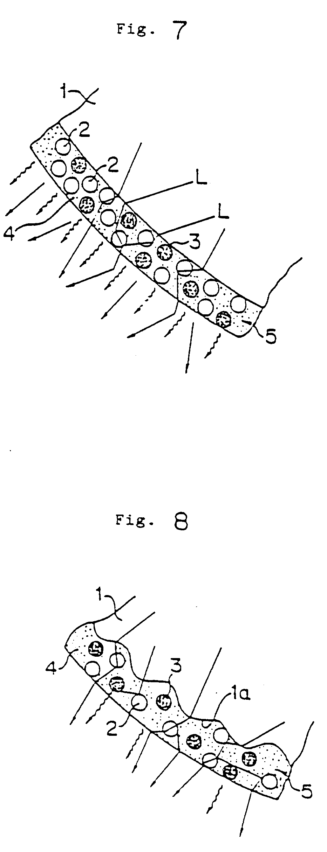

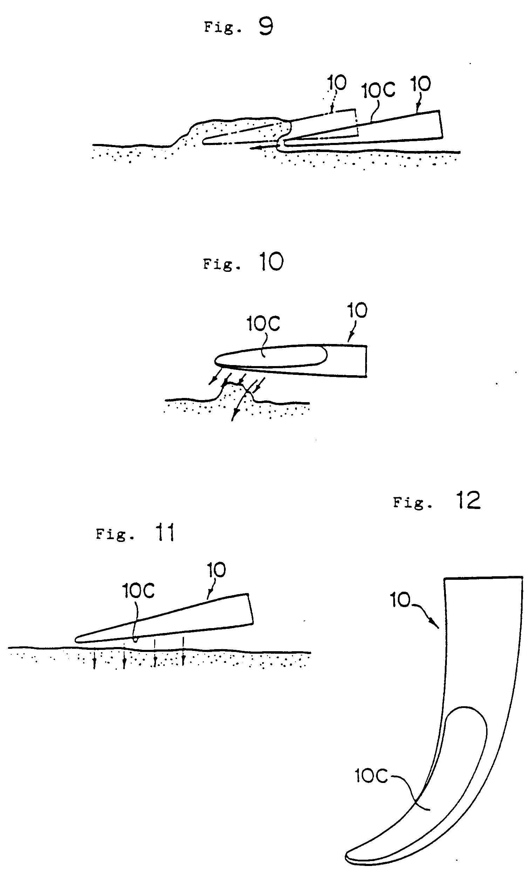

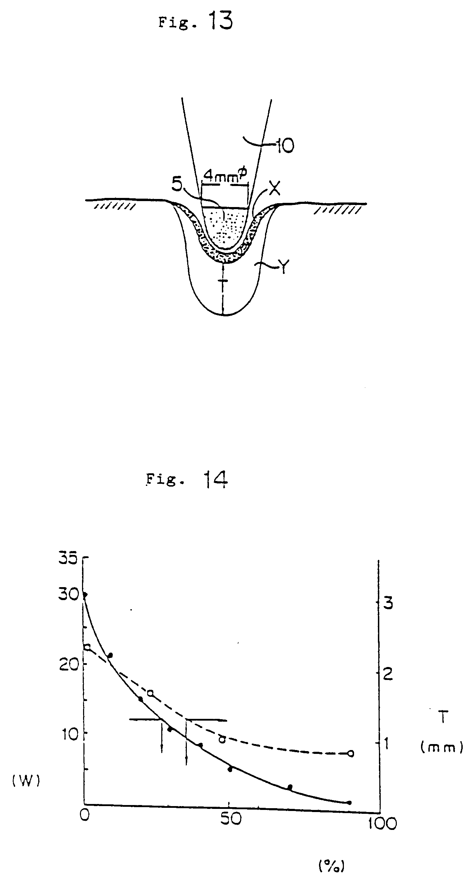

Fig. 1 is a longitudinal sectional view of a probe and a holding member relating to the present invention; Fig. 2 is a front view of a probe; Fig. 3 is a side view thereof; Fig. 4 is transverse sectional views thereof which are shown one by one longitudinally; Figs. 5 and 6 are a side view of another probe and a front view of still another probe; Figs. 7 and 8 are enlarged sectional views of surface layers covering a transmissible member; Figs. 9 - 12 are schematic and perspective illustrations showing operation with a probe for an incision or an exfoliation, operation with the side-edge of a flat part for an incision or an exfoliation, operation with a flat surface for hemostasis and another probe; Fig. 13 is a schematic illustration showing an experiment; Fig. 14 is a graph showing the result of the experiment; Fig. 15 is a front view of a conventional probe.

As shown in Figs. 1, 2, 3 and 4, the probe 10 comprises a cylindrical part 10A at its upper part, substantial conical part 10B at its lower part and two flat surfaces 10C on the both sides of the conical part 10B. The flat surfaces 10C are formed by partial and longitudinal cutting away of the both sides of the conical part 10B. Although the shape of the tip end of the flat surface 10C might be selected properly, in Fig. 2, it is of a substantially semi-circle shape. As shown in Fig. 4, since the conical part 10B is tapered down to the tip end, it is apparent that the distance between the both flat surfaces 10C is gradually shorter to the tip end of the flat surface 10C.

In a modified embodiment, although which is not shown, the probe is provided with an exact conical part at its lower part, then, flat surfaces are formed on the both sides of the exact conical part.

Fig. 5 shows a side view of the probe having another structure. In the lower part of the probe 10, a long and narrow conical shaped flat part, which has two flat surfaces 10C on its both sides, is projected from an upper cylindrical part 10A. Fig. 6 shows a front view of the probe having still another structure. In the lower part of this probe 10, a flat part, which has at least one flat surface 10C and whose upper part is tapered down for distance and whose lower part keeps substantially the same diameter to the end, is projected from a cylindrical part 10A. Therefore, the upper part of the flat surface 10C has substantially an elliptical shape and the lower part of the flat surface 10C, substantially a rectangular shape.

The above mentioned various probes 10 provided with flat surfaces 10C are held by holding members respectively, for example, as shown in Fig. 1.

The cylindrical part 10A of the probe 10 is fitted in a cylindrical female connector 33 and fixed integrally thereto by caulking the mating portions 33a, by using a ceramic type adhesive between the mating surfaces or by the combination of these both means. The female connector 33 has, on the internal surface thereof, a female screw 34 which is adapted to mate removably with male screw 36 of a male connector 35. The female connector 33 has holes 38 which facilitate the passage of cooling water W inside and outside thereof. The holes 38 are disposed adjacent to the top of a light receiving upper part 37 of the probe 10, and for example, oppositely on the circumference of the female connector 33, although only one of them is shown in Fig. 1. On the other hand, the male connector 35 is pressed to be fitted into an end portion of a flexible tube jacket 39 fabricated of, for example, Teflon (the trademark). For this press fitting, the male connector 35 has stepped portions 40 at its base portion in order to be firmly held by the tube jacket 39 so as not to be removed.

A transmitting optical fiber 11 for the laser light is inserted in the tube jacket 39 and the male connector 35. There is a passage 42 between the optical fiber 11 and the tube jacket 39 for passage of cooling water W. The transmitting optical fiber 11 is closely fitted in the tube jacket 39 at its stepped portion 40. Then, the stepped portion 40 has, for example, two slits 40a formed oppositely on a circumference of the stepped portion 40 for letting the cooling water W pass therethrough. A gap 41 for the cooling water W is further provided between the inner surface of the end portion of the male connector 35 and the outer surface of the transmitting optical fiber 11.

The probe held by the above mentioned holding member serves as a laser light emitter while the female connector 33 is connected to mate with the male connector 35. In this case, the emitter is equipped in an endoscope and some other suitable holders. Then, introduced pulse laser light through the transmitting optical fiber 11 is penetrated into the probe 10 from the light receiving base 37, therefore, is emitted from the whole outer surface of the inserting portion 30 of the probe 10. At the same time, the cooling water W is fed through the passage 42, the slit 40a and the gap 41 to cool the probe 10, further, discharged through the opening 38 to flow out on the surface of the living tissues so as to cool them.

Fig. 7 shows an enlarged sectional views of the surface layer 5 covering the above mentioned probes shown in Figs. 1 to 4. The surface layer 5 contains the laser light scattering particles 2 and the laser light absorbing particles 3. Further, as explained before, the laser light transmissible particles are melted to be the transmissible material 4 as the binder. The transmissible material 4 forms layers with the particles in the surface layer 5.

As shown in Fig. 7, the transmissible member 1, which is utilized for forming the probe as its body, is covered with the surface layer 5. Further, as shown in Fig. 8, forming a rough surface 1 a on the transmissible member 1 under the surface layer 5 increases the scattering effect of the laser light.

The transmissible member 1 is preferably fabricated from a natural or artificial ceramic material such as diamond, sapphire, quartz and the like due to their heat resistance.

The light scattering particle, which has a larger refractive index for the laser light than that of the transmissible member, is of a natural or artificial material such as diamond, sapphire, quartz (a melting point is preferably high), single crystal zirconium oxide (Zr₂0₃), high melting point glass, transmissible and heat resistant synthetic resin, laser light reflective metal, and a particle which is laser light reflective or nonreflective metal particle coated with laser light reflective metal such as gold, aluminum and the like by means of the surface treatment such as gilding.

The transmissible material 4 is preferably made from the transmissible particle, which can be melt to form a film and more preferably which has heat resistance, such as natural or artificial sapphire, quartz, glass, transmissible and heat resistant synthetic resin and the like. A suitable transmissible material is selected from these materials in consideration of the relation to the transmissible material 4.

The laser light absorbing particle 3 is made of carbon, graphite, iron oxide, manganese dioxide and the any other materials which can absorb the laser light to generate heat energy.

A content of each particle in the surface layer(wt%) and each average particle size are preferably within ranges shown in a following table respectively. More preferable each content and particle size are put in parentheses.

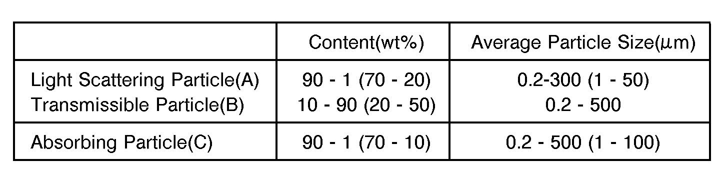

The thickness of the surface layer is preferably 10tim - 5mm, more preferably 30tim - 1 mm. The surface layer is formed by following method. If the surface layer can not be formed to be a desired thickness by one step of the following method, the step should be repeated until a desired thickness can be obtained;

First method; the three kinds of particles are dispersed in a dispersion medium. Then, the medium is heated to a temperature which is higher than the melting point of the transmissible particle.

Finally, the transmissible member is dipped in the heated dispersion.

Second method; the three kinds of particles are melted to be sprayed to the transmissible member.

Further, other suitable methods for forming the surface layer can be used.

By the above mentioned first method, the dispersion dispersing the three kinds of particles can be painted to the transmissible member. Moreover, this painting method is easy, because what should be done is that only a part, which is desired to be covered with the surface layer, of the transmissible member is dipped in the dispersion and therefrom pulled up. Therefore, this method is practical and rational.

As the dispersion medium, suitable liquid such as water, alcohol or mixture of them can be used. Further sugar or starch is added to increase the viscosity of the dispersion medium.

As described before, according to the present invention, the forming of the surface layer 5 on the surface of the transmissible member 1 extends the effective area of the laser light irradiation. Because the laser light is emitted widely in many directions from the surface layer 5.

On the other hand, the inventor performed an experiment as follows, using a conical shaped probe, although which has no flat surface.

In a following section, the content of the light scattering particle, that of the transmissible particle, and that of the laser light absorbing particle will be referred as (A), (B) and (C) respectively. The inventor investigated each change of following two parameters against (C) under fixed condition of (A) : (B) = 2 : 1. One investigated parameter is a laser light power level with which an incision to a pig's liver can be started. Another parameter is the depth T of a coagulation layer Y below a carbonized layer X of treated tissues (T, Y and X are shown in Fig. 13). Then, from the result shown in Fig. 14, following things are known.

According to the relation of the power level and the percentage of (C), when the percentage of (C) is high, the incision can be started with the low power level of the laser light, then it is possible for the probe to be moved quickly. Then, according to the relation of the depth T and the percentage of (C), as the percentage of (C) is increased, the depth T is decreased. Since the effect of hemostasis can be known by the depth T, the hemostasis of the treated tissues is turned out to be reduced as the percentage of (C) is increased.

Therefore, the probe with high percentage of (C) in the surface layer can be used effectively for the incision to the tissues which bear the damage to some extent such as skin, fat layer and the like.

On the other hand, the probe with low percentage of (C) can be used efficiently for incision to the tissue, for which the hemostasis is regarded to be important. This type of the tissues is, for example, liver, heart and the like. In this case, it is clear that the laser light power level of the output from a laser light generator must be raised and the probe must be moved slowly.

Referring to this experiment and the like, the inventor introduced these two equations, (1) and (2).

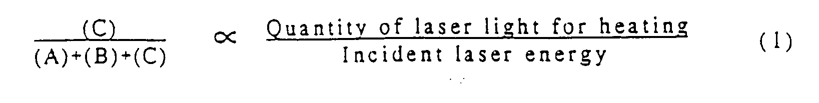

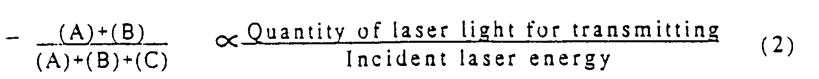

Equation (1) means that heat generation is progressed as (C) is increased. Accordingly, under high percentage of (C), an incision is carried out by mainly vaporization. Therefore, since most of the incident laser light energy is spent for the heating, the laser light can not penetrate so deeply into the tissues. As a result, the depth of the coagulation layer is reduced.

Equation (2) means that penetration of the laser light into the tissues is progressed as (C) is decreased. Accordingly, under the low percentage of (C), the tissues absorbing laser light are heated, thus, the coagulation is made in the tissues.

It is needless to say, these results correspond to the result of the above mentioned experiment shown in Fig. 14.

Therefore, if some kinds of probes which differs in only percentage of (C) in the surface layers are prepared in advance, a suitable probe can be selected in accordance with a medical purpose, thereby a suitable treatment can be carried out easily. Further, according to the present invention, since the probe 10 is provided with at least one flat surface, much more treatments can be carried out.

Now, operation of the probe 10 of the present invention will be explained referring Figs. 9, 10 and 11.

At first, by the probe 10 of the present invention, the tissues can be incised or exfoliated. In this case, as shown in Fig. 9, the probe 10 is moved to the left or in a direction indicated by an arrow in this figure and/or is swung in a transverse direction to the moving direction. As another method, the probe 10 can be placed vertically to the surface of the tissues; the probe 10 stands up for incising the tissues.

Second, as shown in Fig. 10, a tumor and the like produced on the tissues can be cut away or exfoliated by the both side-edges of the flat part moved in a direction indicated by arrows in this figure.

Further, as shown in Fig. 11, hemostasis for an ulcerated part can be carried out by laser light irradiation with small energy from the flat surface 10C facing the ulcerated part.

Fig. 12 shows another embodiment of the probe 10, the lower part of which is bent with respect to the upper part. In this probe 10, at least one flat surface 10C is formed on the bending fore end portion. By this probe 10, another type of effective area of the laser light irradiation can be obtained.

It will be appreciated from the foregoing description that, according to the present invention, with one laser light transmitting probe, treatments such as an exfoliation and hemostasis as well as an incision and vaporization can be performed efficiently and selectively corresponding to the conditions of each target area of living tissues.

## Claims

1. A laser light transmitting probe (10) comprising an upper part into which laser light is impinged, and a lower part (10B) from which said laser light is emitted, at least one flat surface (10C) being formed on said lower part by partial and longitudinal cutting away of one side of the lower part (10B) and having two side edges, characterized by the fact that a surface layer (5), containing laser light absorbing particles (3) and laser light scattering particles (2) having a larger refractive index than that of the material of said probe (10), is formed on said at least one flat surface (10C).

2. A laser light transmitting probe according to claim 1, wherein said lower part is provided with a conical part (10B).

3. A laser light transmitting probe according to claim 1, wherein said probe (10) is symmetric with respect to the axis of said probe.

4. A laser light transmitting probe according to claim 1, wherein said lower part (10B) is bent with respect to the upper part.

5. A laser light transmitting probe according to claim 1, wherein a binder (4), made from a laser light transmissible material, is formed on said at least one flat surface (10C).

6. A laser light transmitting probe according to claim 1 or 5, wherein said surface layer (5) is provided on a rough surface (1 a) formed on said at least one flat surface (10C).

## Patentansprüche

1. Laserstrahlsonde (10), umfassend einen oberen Teil, in den Laserlicht einfällt, und einen unteren Teil (10B), von dem dieses Laserlicht ausgesendet wird, mindestens eine flache Oberfläche (10C), die auf dem unteren Teil durch das teilweise und in Längsrichtung erfolgende Wegschneiden einer Seite des unteren Teils (10B) ausgebildet ist und zwei Seitenkanten aufweist, dadurch gekennzeichnet, daß eine Oberflächenschicht (5), die Laserlicht absorbierende Partikel (3) und Laserlicht streuende Partikel (2) mit einem größeren Brechungsindex als das Material der Sonde (10) enthält, auf dieser mindestens einen flachen Oberfläche (10C) ausgebildet ist.

2. Laserstrahlsonde nach Anspruch 1, worin der untere Teil mit einem konischen Teil (10B) versehen ist.

3. Laserstrahlsonde nach Anspruch 1, worin die Sonde (10) in bezug auf die Achse dieser Sonde symmetrisch ist.

4. Laserstrahlsonde nach Anspruch 1, worin der untere Teil (10B) in bezug auf den oberen Teil gebogen ist.

5. Laserstrahlsonde nach Anspruch 1, worin ein Bindemittel (4) aus einem laserlichtdurchlässigen Material auf der mindestens einen flachen Oberfläche (10C) ausgebildet ist.

6. Laserstrahlsonde nach Anspruch 1 oder 5, worin die Oberflächenschicht (5) auf einer rauhen Oberfläche (1 a) vorgesehen ist, welche auf der mindestens einen flachen Oberfläche (10C) ausgebildet ist.

## Revendications

1. Sonde (10) de transmission de lumière laser comprenant une partie supérieure dans laquelle pénètre la lumière laser et une partie inférieure (10B) d'où est émise la lumière laser, au moins une surface plane (10C) étant ménagée sur ladite partie inférieure par découpe partielle et longitudinale d'un côté de la partie inférieure (10B) et pourvue de deux bords latéraux, caractérisé par le fait qu'il est formée sur ladite surface plane (10C) une couche superficielle (5), contenant des particules (3) absorbant la lumière laser et de particules (2) diffusant la lumière laser ayant un indice de réfraction plus grand, que celui du matériau de la sonde (10).

2. Sonde de transmission de lumière laser selon la revendication 1, dans laquelle ladite partie inférieure est pourvue d'une partie conique (10B).

3. Sonde de transmission de lumière laser selon la revendication 1, dans laquelle la sonde (10) est symétrique par rapport à son axe.

4. Sonde de transmission de lumière laser selon la revendication 1, dans laquelle la partie inférieure (10B) est recourbée par rapport à la partie supérieure.

5. Sonde de transmission de lumière laser selon la revendication 1, dans laquelle un liant (4), formé d'un matériau transmettant la lumière laser, est formé sur ladite surface plane (10C).

6. Sonde de transmission de lumière laser selon la revendication 1 ou 5, dans laquelle la couche superficielle (5) est formée sur une surface rugueuse (1 a) ménagée sur ladite surface plane (10C).
